# EUROPEAN PATENT APPLICATION

(11) **EP 4 663 775 A1**
(43) Date of publication of application: **17.12.2025**
(21) Application number: 24753644.4
(22) Date of filing: 07.02.2024
(51) Int. Cl.: C12Q 1/6886, G01N 33/574

(54) **P21-ACTIVATED KINASE 4 INHIBITION AND E-CADHERIN EXPRESSION INDUCER AND SCREENING METHOD THEREFOR**

(30) Priority: 08.02.2023 KR 20230016676; 04.10.2023 KR 20230131631
(71) Applicant: Seoul National University Hospital, Seoul 03080 (KR)
(72) Inventor: KIM, Jin Won, Seongnam-si, Gyeonggi-do 13629 (KR); KIM, Kui-Jin, Seongnam-si, Gyeonggi-do 13603 (KR); HWANG, Sung-Hyun, Seongnam-si, Gyeonggi-do 13568 (KR); SUNG, Ji Hea, Suwon-si, Gyeonggi-do 16512 (KR); NAM, Milang, Yongin-si, Gyeonggi-do 16909 (KR)
(74) Representative: V.O.
(86) International application number: PCT/KR2024/001859
(87) International publication number: WO 2024/167323

(57) **Abstract**

The present specification pertains to a method for screening a p21-activated kinase (PAK4) inhibition and E-cadherin expression inducer using the novel mechanism of PAK4's regulation of E-cadherin expression and to a PAK4 inhibitor and E-Cadherin expression inducer, according to the method, useful for the prevention, treatment, or metastasis inhibition of cancer, particularly pancreatic cancer. Through the novel mechanism by which PAK4 regulates E-Cadherin expression, it is possible to develop targeted therapies for cancer or metastatic cancer effectively. Particularly provided can be first-in-class innovative new drugs in the field of pancreatic cancer or metastatic cancers derived from pancreatic cancer, where there is currently an unmet need due to the lack of effective treatments.

## Description

### Technical Field

The present specification pertains to a method for screening a p21-activated kinase 4 (PAK4) inhibition and E-cadherin expression inducer using the novel mechanism of PAK4's regulation of E-cadherin expression and to a therapy, according to the method, useful for the prevention, treatment, or metastasis inhibition of cancer, particularly pancreatic cancer.

### [National Research and Development Program Supporting the Invention]

Unique Project No. 1711164170
Project No. 2020R1F1A1076372
Ministry: Ministry of Science and ICT
Project Management Agency: National Research Foundation of Korea
Research Program Title: Individual Basic Research (Ministry of Science and ICT, R&D)
Research Project Title: Study on the Metastatic Mechanisms involving p21-Activated Kinase 4 in Pancreatic Cancer
Contribution Ratio: 1/1
Seoul National University Bundang Hospital
Research Period: March 1, 2022, to February 28, 2023

### Background

When developing new drugs, all clinical studies for evaluating the efficacy of drugs are mainly conducted using in vitro cell experiments and animal experiments. In the evaluation of drug efficacy using cells, the drug efficacy may be evaluated by treating cells with drug candidate materials and observing the astrology of the cells, staining, polymerase chain reaction (PCR), protein analysis, and the like. Metabolomics is the study for comprehensive research on the entire metabolome created during the cellular process. This enables observation of the overall cell state, metabolic state related to the system, and macroscopic biochemical events, and derivation of biomarkers related to drug efficacy. The current biomarker market based on omics includes all genomes, protein bodies, and metabolomes, but mainly focuses on the genomes. However, it is necessary to develop a new drug development screening system using a metabolome that can confirm the actual drug efficacy as a substance close to the expression trait of the living body.

According to a recent report by the World Health Organization, pancreatic cancer is a fatal cancer with the highest mortality rate among all cancers. Since pancreatic cancer is an indolent tumor with a rapid progression from diagnosis to death, the majority of pancreatic cancer patients have already metastasized at the time of diagnosis, making it very difficult to treat the cancer. Although the basic treatment for pancreatic cancer is radical resection, only 10 to 20% of patients can receive the basic treatment of radical resection according to the progression of pancreatic cancer. Even after the surgery, many patients have a very low 5-year survival rate of around 10% due to local recurrence or metastasis.

To this date, gemcitabine/nab-paclitaxel or 5-fluorouracil, folinic acid [leucovorin], irinotecan, oxaliplatin (FOLFIRINOX) has been provided as the primary standard therapy for pancreatic cancer, but the median survival has not exceeded 12 months.

### Detailed Description of the Invention

### Technical Solution

An exemplary implementation of the present invention provides a method for efficiently developing targeted therapies for cancer, particularly pancreatic cancer, or metastatic cancer, particularly metastatic cancer derived from pancreatic cancer, through the novel mechanism by which PAK4 regulates E-cadherin expression, and a targeted therapy according to the method.

### Summary of the Invention

In one aspect, exemplary implementations of the present invention provide a method for screening a p21-activated kinase 4 (PAK4) inhibition and E-cadherin expression inducer, the method comprising treating cells where PAK4 is overexpressed with a candidate substance, measuring the amount of E-cadherin expression in the cells where PAK4 is overexpressed after the candidate substance treatment, and comparing the measured amount of E-cadherin expression with the amount of E-cadherin expression before the candidate substance treatment.

In another aspect, exemplary implementations of the present invention provide a composition for screening the PAK4 inhibition and E-cadherin expression inducer.

In another aspect, exemplary implementations of the present invention provide a kit for screening the PAK4 inhibition and E-cadherin expression inducer, comprising the composition for screening the PAK4 inhibition or E-cadherin expression inducer described above.

In another aspect, exemplary implementations of the present invention provide the PAK4 inhibition and E-cadherin expression inducer or a composition for preventing or treating cancer comprising the same as an active ingredient.

### Effects of the Invention

According to exemplary implementations of the present invention, with the novel mechanism by which PAK4 regulates E-cadherin expression, targeted therapies for cancer or metastatic cancer can be effectively developed. Particularly provided can be first-in-class innovative new drugs in the field of pancreatic cancer or metastatic cancers derived from pancreatic cancer, where there is currently an unmet need due to the lack of effective treatments.

### Brief Description of the Drawings

FIGS. 1A to 1C show the results of evaluating the prognosis of a PAK4 overexpression group and metastasis-related genes through TCGA data analysis.
FIGS. 2A to 2E show the results of evaluating PAK4 expression and cell invasion in pancreatic cancer cell lines.
FIG. 3A to FIG. 3F show the results of analyzing cell migration, invasion, displacement, and metastatic three-dimensional morphology caused by PAK4 knockdown in cells where PAK4 is overexpressed.
FIGS. 4A to 4E show the results of analyzing the effects of PAK4 knockdown on epithelial-mesenchymal transition-regulating transcription factors and protein expression.
FIGS. 5A to 5G show the results of analyzing the effects of PAK4 on the transcription and translation of E-cadherin.
FIGS. 6A to 6G show the results of analyzing expression and binding of PAK4, CDC42, and E-cadherin at the same position.
FIGS. 7A to 7E show the results of analyzing the decrease in cell migration, invasion, and displacement due to increased PAK4 overexpression and CDC42 activity and decreased E-cadherin.
FIGS. 8A to 8J show the results of evaluating the effects of PAK4 knockdown on the growth and metastasis of primary cancer in a pancreatic cancer orthotopic model.

### Best Mode for Implementing the Invention

Hereinafter, exemplary implementations of the present invention will be described in more detail.

The implementations and embodiments of the present invention disclosed herein are merely illustrative for the purpose of description. The implementations and embodiments may be embodied in various forms and shall not be construed as being limited to the implementations and the embodiments described herein. Since the present invention can have various modifications and various forms, it should be understood that these implementations and embodiments are not intended to limit the present invention to the specific disclosed forms, but include all modifications, equivalents, and alternatives falling within the spirit and technical scope of the present invention.

Surprisingly, it was found by the present inventors that inhibiting p21-activated kinase 4 (PAK4) in cancer cells directly regulates the E-cadherin signaling mechanism, thereby inhibiting the cell migration, invasion, and displacement of cancer cells.

In detail, the present inventors confirmed that when PAK4 is inhibited in cancer cells, the activity of cell division cycle 42 or cell division control protein 42 homolog (CDC42) is inhibited and E-cadherin is increased, thereby reducing the cell migration, invasion, and displacement of cancer cells. It was confirmed that expressions of PAK4 and E-cadherin are negatively correlated and the mechanism by which PAK4 regulates E-cadherin is involved in both transcription and translation processes. In addition, it was confirmed that "PAK4-CDC42-E-cadherin" are expressed at the same position in cells, and proteins are directly bound thereto. During this process, it was further confirmed that PAK4 controls the safety of E-cadherin protein through CBLL1/Hakai ubiquitinase regulation. In addition, in particular, it was confirmed that PAK4 and E-cadherin are negatively correlated in pancreatic cancer cells, and that cancer metastasis can be suppressed when PAK4 in pancreatic cancer cells is targeted and PAK4 overexpression is suppressed.

Thus, in one aspect, exemplary implementations of the present invention provide a method for developing innovative new drugs based on the novel mechanism described above, the method comprising treating cells where PAK4 is overexpressed with a candidate substance, measuring the amount of E-cadherin expression in the cells where PAK4 is overexpressed after the candidate substance treatment, and comparing the measured amount of E-cadherin expression with the amount of E-cadherin expression before the candidate substance treatment.

The PAK4 refers to a protein encoded by the human PAK4 gene, and PAK4 inhibition refers to inhibiting PAK4 overexpression by targeting PAK4. In addition, the candidate substance refers to a candidate substance for the PAK4 inhibition and E-cadherin expression inducer.

The overexpression may mean, but is not limited to, that the expression of PAK4 is 1.5 times or more, 1.6 times or more, 1.7 times or more, 1.8 times or more, 1.9 times or more, 2.0 times or more, 2.5 times or more, 3 times or more, 3.5 times or more, 4 times or more, 4.5 times or more, 5 times or more, 10 times or more, 15 times or more, 20 times or more, 25 times or more, 30 times or more, 35 times or more, 40 times or more, 45 times or more, 50 times or more than that of normal cells.

In embodiments, the treatment may include any treatment method for inducing the expression of a particular protein in a particular cell, known to those skill in the art.

The E-cadherin refers to a protein encoded by the human E-cadherin gene.

The amount of E-cadherin expression includes any objective or relative value of the concentration or mass of E-caedherin contained in the sample and is not limited to the type of the value.

The comparison includes, but is not limited to, any method for relatively comparing the amounts of the particular protein expressions in the particular cells, known to those skilled in the art, and is not limited to the type of the method.

The screening refers to PAK4 inhibition and zero selection and/or detection of a particular candidate substance or component and is not limited to the method and subject.

In one embodiment, after the comparing step, the method may further include determining that the candidate substance is the PAK4 inhibition and E-cadherin expression inducer when the measured amount of E-cadherin expression is greater than the amount of E-cadherin expression before the candidate substance treatment.

Specifically, the degree of increase in the amount of E-cadherin expression in the cells where PAK4 is overexpressed after the candidate substance treatment varies depending on the degree of inhibition of the PAK4 activity by the candidate substance, and thus is not limited to the degree of increase therein as long as it is statistically significant. For example, the degree of increase in the amount of E-cadherin expression in the cells where PAK4 is overexpressed after the candidate substance treatment may be 120% or more, 130% or more, 140% or more, 150% or more, 160% or more, 170% or more, 180% or more, 190% or more, 200% or more, 210% or more, 220% or more, or 230% or more.

In one embodiment, the cells where PAK4 is overexpressed may be cancer cells. For example, the cancer cells may include cerebrospinal tumor cells, head and neck cancer cells, lung cancer cells, breast cancer cells, thymoma cells, mesothelioma cells, esophageal cancer cells, gastric cancer cells, colorectal cancer cells, liver cancer cells, biliary tract cancer cells, kidney cancer cells, bladder cancer cells, prostate cancer cells, testicular cancer cells, germinoma cells, ovarian cancer cells, cervical cancer cells, endometrial cancer cells, lymphoma cells, acute leukemia cells, chronic leukemic cells, multiple myeloma cells, sarcoma cells, malignant melanoma cells, skin cancer cells, or pancreatic cancer cells.

In one embodiment, the cells where PAK4 is overexpressed may be, in particular, pancreatic cancer cells. For example, the pancreatic cancer cells may be from a pancreatic cancer cell line, wherein the pancreatic cancer cell line may include SNU410, SNU213, CAPAN-2, CAPAN-1, ASPC-1, MIAPaCa-2, Su8686, PANC1, PATU-8988S or PATU-89 88T.

In one embodiment, the cells where PAK4 is overexpressed may be metastatic cancer cells. The metastatic cancer cells may be related to epithelial-mesenchymal transition (EMT). For example, the metastatic cancer cells are EMT cells or EMT cell line. In addition, the metastatic cancer cells may be cancer cells before, during, or after the conversion of primary cancer cells into circulating tumor cells (CTCs). The cancer cells are the same as those described above.

In one embodiment, the metastatic cancer cells are derived from pancreatic cancer cells. The pancreatic cancer cells may be derived from a pancreatic cancer cell line as described above, wherein the pancreatic cancer cell line may include SNU410, SNU213, CAPAN-2, CAPAN-1, ASPC-1, MIAPaCa-2, Su8686, PANC1, PATU-8988S, or PATU-8988T.

In one embodiment, the candidate substance may be an anticancer drug. The anticancer drug may include both a targeted therapy for cancer and a targeted therapy for metastatic cancer.

For example, the anticancer drug may be a small molecule drug, a protein, a PROTAC compound, a peptide, peptide mimetics, synthetic peptides or synthetic oligonucleotides, DNA, mRNA, small interfering RNA, small hairpin RNA, micro RNA, PNA, or foldamers.

In one embodiment, the anti-cancer drug may be the small molecule drug.

In one embodiment, before the treating of cells with the candidate substance, the method may further include overexpressing PAK4 in PAK4 wild-type cells.

In one embodiment, the method may further include measuring the CDC42 activity in the cells where PAK4 is overexpressed after the candidate substance treatment and comparing the same with the activity before the candidate substance treatment, and determining that the candidate substance is the PAK4 inhibition and E-cadherin expression inducer if the measured CDC42 activity in the cells is less than the activity before the candidate substance treatment.

Specifically, the degree of decrease in the CDC42 activity in the cells where PAK4 is overexpressed after the candidate substance treatment varies depending on the degree of inhibition of the PAK4 activity by the candidate substance and is not limited to the degree of decrease therein as long as it is statistically significant. For example, the degree of decrease in the CDC42 activity in the cells where PAK4 is overexpressed after the candidate substance treatment may be 90% or less, 80% or less, 70% or less, 60% or less, 50% or less, 40% or less, 30% or less, or 20% or less.

The CDC42 refers to a protein encoded by the human CDC42 gene.

In one embodiment, the method may further include measuring a change in the displacement of the cells where PAK4 is overexpressed after the candidate substance treatment and comparing the measured change with the displacement before the candidate substance treatment; and determining that the candidate substance is the PAK4 inhibition and E-cadherin expression inducer when the measured displacement of the cells is less than the displacement before the candidate substance treatment.

Specifically, the degree of decrease in the displacement of the cells where PAK4 is overexpressed after the candidate substance treatment varies depending on the degree of inhibition of the PAK4 activity by the candidate substance and is not limited to the degree of decrease therein as long as it is statistically significant. For example, the degree of decrease in the displacement of the cells where PAK4 is overexpressed after the candidate substance treatment may be 90% or less, 80% or less, 70% or less, 60% or less, 50% or less, 40% or less, 30% or less, or 20% or less.

In one embodiment, the method may further include measuring a change in the amount of gene expression in the cells where PAK4 is overexpressed, by one or more of Zeb1, Zeb2, Snail, Slug, Twist and Vimentin, after the candidate substance treatment and comparing the same with the amount of gene expression before the candidate substance treatment; and determining that the candidate substance is the PAK4 inhibition and E-cadherin expression inducer when the measured amount of gene expression in the cells is less than the amount of gene expression before the candidate substance treatment.

Specifically, the degree of decrease in the amount of gene expression in the cells where PAK4 is overexpressed after the candidate substance treatment varies depending on the degree of inhibition of the PAK4 activity by the candidate substance and thus is not limited to the degree of decrease therein as long as it is statistically significant. For example, the degree of decrease in the amount of gene expression in the cells where PAK4 is overexpressed after the candidate substance treatment may be 90% or less, 80% or less, 70% or less, 60% or less, 50% or less, 40% or less, 30% or less, or 20% or less.

In another aspect, exemplary implementations of the present invention provide a composition for screening the PAK4 inhibition and E-cadherin expression inducer and/or a kit comprising the same.

In one embodiment, the composition may include a substance that detects E-cadherin and/or CDC42 or a substance that measures the amount of expression thereof. The substance that detects E-cadherin and/or CDC42 or the substance that measures the amount of expression thereof may be a known substance and is not particularly limited thereto.

In one embodiment, the composition may include cells where PAK4 is overexpressed. The cells where PAK4 is overexpressed may be metastatic cancer cells as described above, and the metastatic cancer cells may be associated with EMT. For example, the metastatic cancer cells may be cells that are undergoing EMT or cells that have undergone EMT. The metastatic cancer cells also may be cancer cells before, during, or after the conversion of primary cancer cells into CTCs.

In one embodiment, the kit may further include instructions including the method for screening the PAK4 inhibition and E-cadherin expression inducer. For example, the instructions may be in the form of a pamphlet or leaflet or in digital form. The instructions may include a description on a label affixed to the kit and a surface of the package including the kit. The instructions may further include guidance including information published or provided via an electrical medium, such as the Internet.

In another aspect, exemplary implementations of the present invention provide a pharmaceutical composition for preventing or treating cancer, comprising the PAK4 inhibition and E-cadherin expression inducer as an active ingredient.

In one embodiment, the PAK4 inhibition and E-cadherin expression inducer may increase the amount of E-cadherin expression in the cells where PAK4 is overexpressed.

In one embodiment, the PAK4 inhibition and E-cadherin expression inducer may decrease the amount of CDC42 expression and increase the amount of E-cadherin expression in the cells where PAK4 is overexpressed. That is, the PAK4 inhibition and E- cadherin expression inducer may be a PAK4 inhibition, CDC42 inhibition and E-cadherin expression inducer.

In one embodiment, the PAK4 inhibition and E-cadherin expression inducer may be the above-mentioned anticancer drugs, such as small molecule drugs, proteins, PROTAC compounds, peptides, peptide mimetics, synthetic peptides or synthetic oligonucleotides, DNA, mRNA, small interfering RNA, small hairpin RNA, micro RNA, PNA, or foldamers.

In one embodiment, the cancer may be, in particular, pancreatic cancer, but is not limited thereto. For example, the cancer may include cerebrospinal tumor, head and neck cancer, lung cancer, breast cancer, thymoma, mesothelioma, esophageal cancer, stomach cancer, colon cancer, liver cancer, biliary tract cancer, kidney cancer, bladder cancer, prostate cancer, testicular cancer, germ cell tumor, ovarian cancer, cervical cancer, endometrial cancer, lymphoma, acute leukemia, chronic leukemia, multiple myeloma, sarcoma, malignant melanoma, skin cancer, or pancreatic cancer.

In one embodiment, the cancer may be metastatic cancer. For example, the metastatic cancer may be derived from cells where the cancer is undergoing or has undergone EMT.

In one embodiment, the pharmaceutical composition may be a cancer metastasis inhibitor that inhibits cancer metastasis.

In one embodiment, the pharmaceutical composition may be administered orally, parenterally, rectally, topically, transdermally, intravenously, intramuscularly, intraperitoneally, or subcutaneously. The dosage form for oral administration may be, but is not limited to, tablets, pills, soft and hard capsules, granules, powders, fine granules, solutions, emulsions, or pellets. The dosage form for parenteral administration may be, but is not limited to, solutions, suspensions, emulsion, gels, injections, drops, suppositories, patches or sprays. The dosage form may be easily prepared according to conventional methods in the art and may further include surfactants, excipients, humectants, emulsifiers, suspensions, salts or buffers for regulating osmotic pressure, colorants, flavoring agents, stabilizers, preservatives, storage agents, or other commonly used adjuvants.

The active ingredient of the pharmaceutical composition according to one embodiment of the present invention may vary depending on the age, sex, weight, pathology and severity thereof of the subject, the route of administration, or the judgment of the prescriber.

Hereinafter, the present invention will be described in more detail by using the embodiments. It will be obvious to those skilled in the art that these embodiments merely illustrate the present invention, and the scope of the present invention shall not be construed as being limited by these embodiments.

### Embodiments

### [Embodiment 1] Genetic Analysis of Prognosis and Metastasis in Pancreatic Cancer Patient Group with PAK4 Overexpression Using TCG Data Analysis

Experiment Method: To evaluate the effect of PAK4 amplification on disease prognosis in PDAC patients, publicly available TCGA data at cBioportal (https://www.cbioportal.org/) were analyzed.

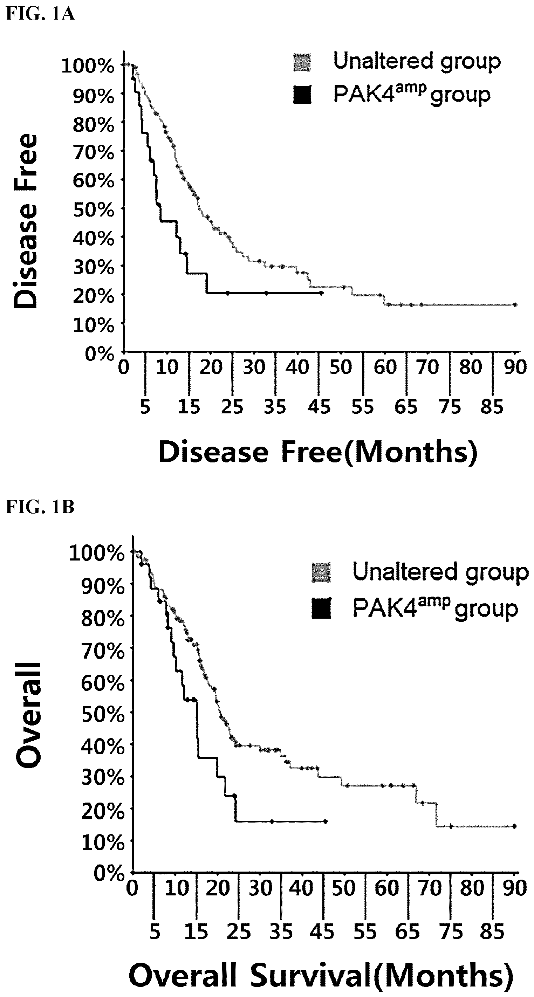

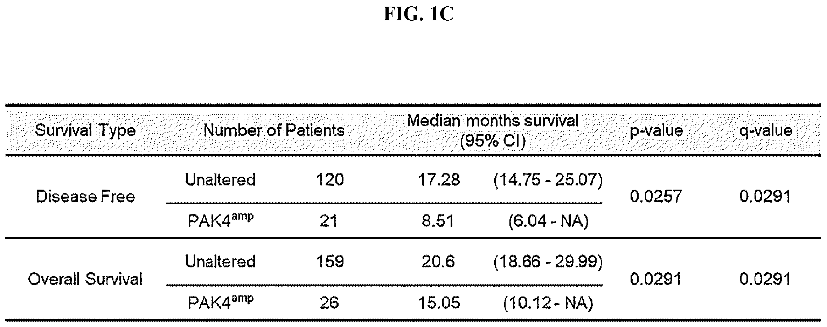

Experiment Result: According to the results of TCGA PanCancer Atlas and UTSW Nat Commun analysis, about 12% of patients diagnosed with pancreatic cancer were found with PAK4 overexpression, and the median survival rate of the PAK4 wild-type patients was 17.28 months, while the median survival rate of the patients with PAK4 overexpression was 8.51 months, which was about 51% shorter than that of the PAK4 wild-type patients. The overall survival rate of the PAK4 wild-type patients was 20.6 months, while the overall survival rate of the patients with PAK4 overexpression was 15.05 months, which was 27% shorter than that of the PAK4 wild-type patients (FIGS. 1A to 1C, p=0.0257, and p=0.00291).

### [Embodiment 2] Evaluation of PAK4 Expression and Cell Invasion in Pancreatic Cancer Cell Line

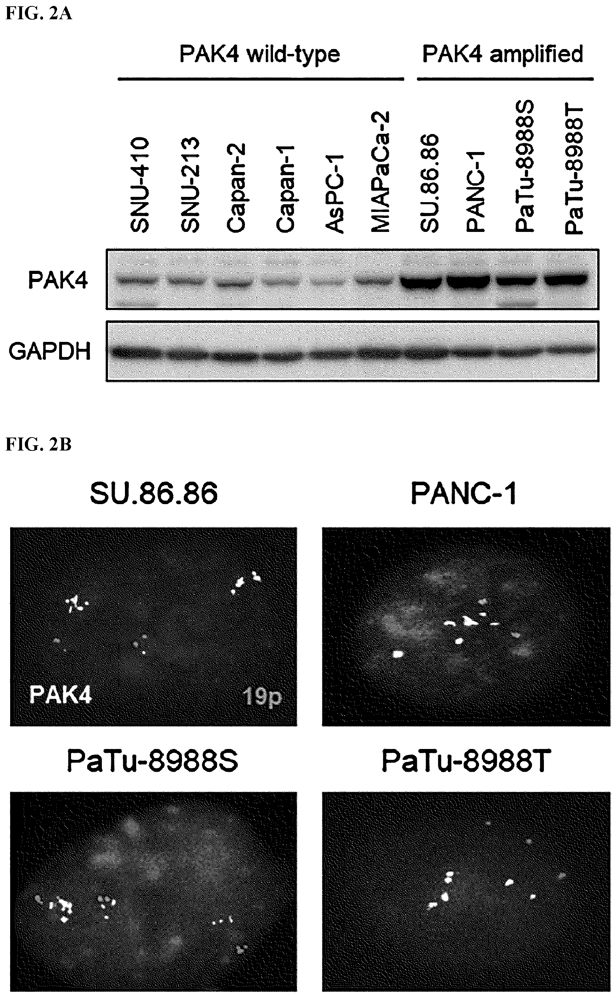

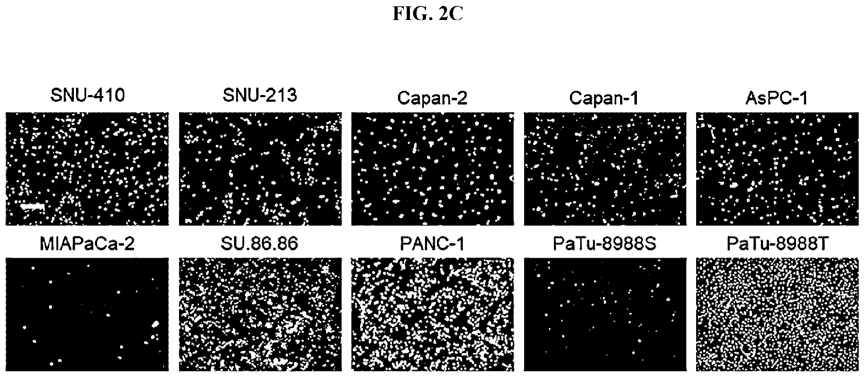

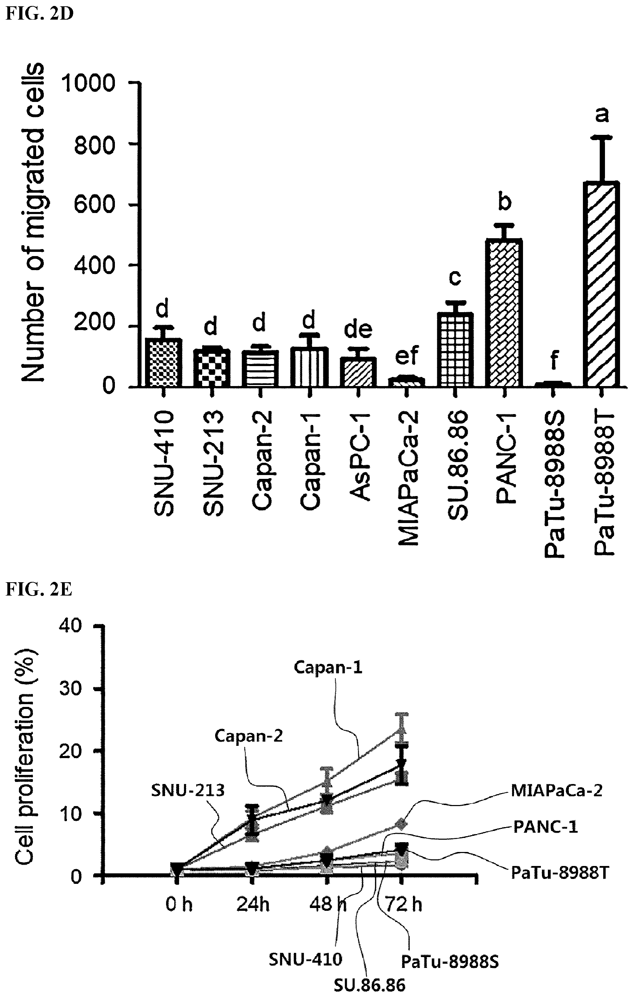

Experiment Method: The amount of PAK4 protein expression was assessed in SNU-410, SNU-213, Capan-2, Capan-1, AsPC-1, MIAPaCa-2, SU.86.86, PANC-1, PaTu-8988s, and PaTu-8888T. GAPDH was used as a protein loading control (FIG. 2A). Fluorescence in situ hybridization (FISH) using probes within the amplified genetic region was performed using probes labeled with Cy3 (red) and centromere reference probes labeled with FITC (green) (FIG. 2B). Cells derived from specific PDAC cells were measured for overnight migration. The experiment was repeated three times, and the data was expressed as mean ± standard deviation (FIGS. 2C and 2D). Cell proliferation analysis of PAK4 wild-type cells (SNU-410,SNU-213, Capan-2, Capan-1, AsPC-1, and MIAPaCa-2) and PAK4-amplified cells (SU.86.86, PANC-1, PaTu-8988s, and PaTu-8988T) was performed using the CellTiter-Glo test and compared the cell lines. The experiment was repeated three times, and data was expressed as means ± standard deviation (FIG. 2E).

Experiment Result: It was hypothesized that PAK4 overexpression is closely related to metastasis. To verify the hypothesis, the basal amount of PAK4 protein expression and the PAK4 amplification were evaluated using 10 pancreatic cancer cell lines (FIG. 2A and FIG. 2B). The PAK4 amplification was shown in the cells where PAK4 is overexpressed. In addition, as a result of evaluating whether PAK4 overexpression affects cell migration, Su8686, PANC1, and PATU-8988T were found to have the highest cell migration capacity (FIGS. 2C and 2D). In addition, it was observed that these functions are not significantly related to cell growth (FIG. 2E).

### [Embodiment 3] Comparison of Three-Dimensional Morphological Analysis of Cell Migration, Displacement, and Metastasis Due to PAK4 Knockdown in Cells where PAK4 is Overexpressed

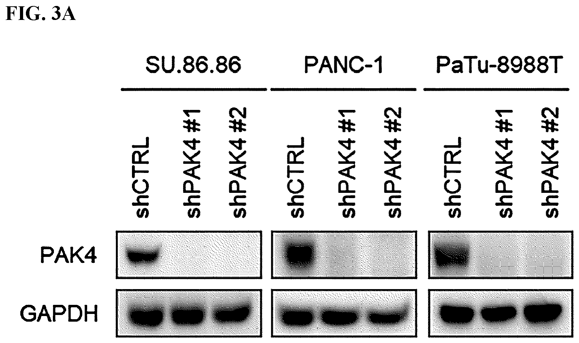

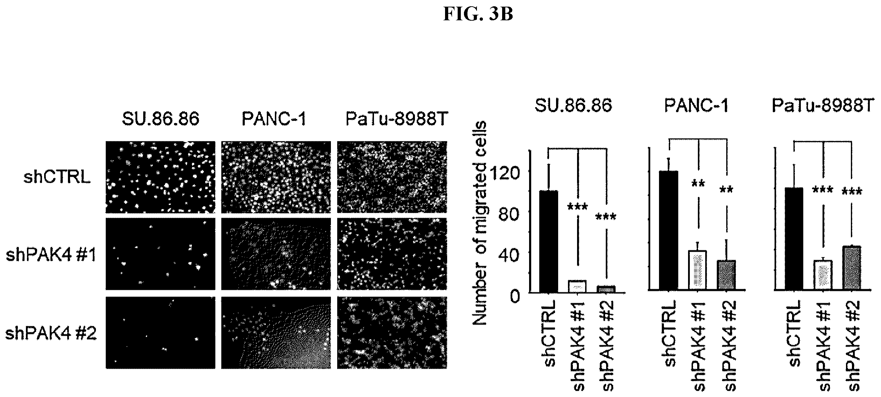

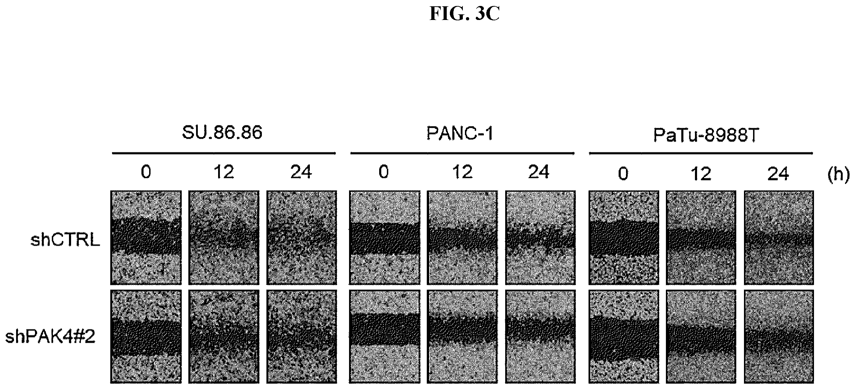

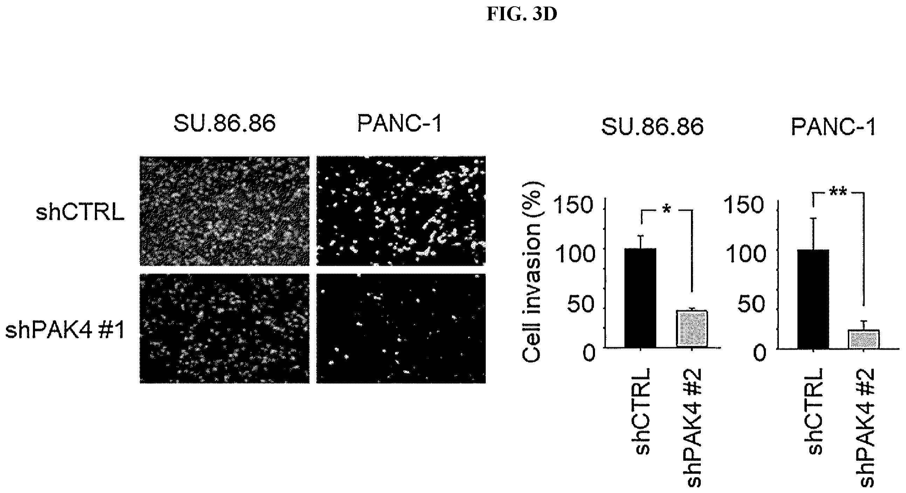

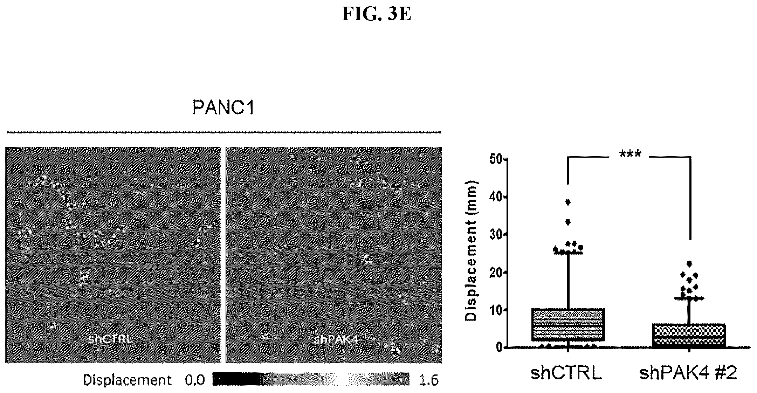

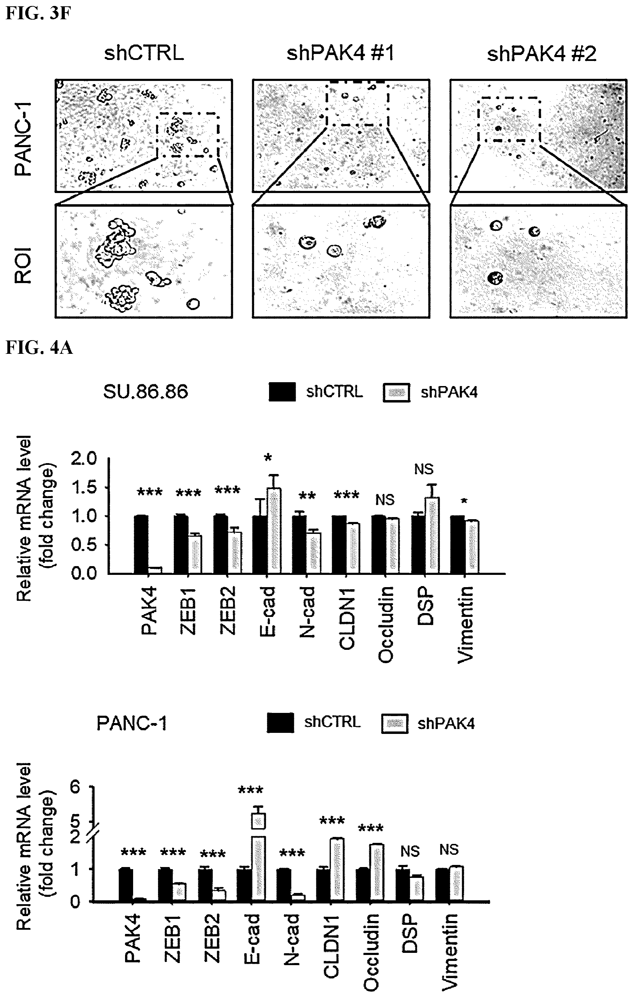

**Experiment Method:** A cell line in which PAK4 expression was stably inhibited was developed using shRNA in SU.86.86, PANC-1 and PaTu-8988T cells (FIG. 3A). A single cell migration evaluation method was used to evaluate the effect of PAK4 inhibition on migration of SU.86.86, PANC-1, and PaTu 8988T cells (FIG. 3B). A group cell migration evaluation method was used to evaluate cell migration (FIG. 3C). Matrigel-coated transwell analysis was used to evaluate the effect of PAK4 inhibition on invasion of SU.86.86 and PANC-1 cells (FIG. 3D). The cell displacement was analyzed through IncuCyte imaging in PANC-1 cells using shCTRL or shPAK4 (FIG. 3E). Three-dimensional cell growth capacity due to PAK4 inhibition was evaluated (FIG. 3F).

**Experiment Result:** In this experiment, PAK4 knockdown was performed using two shRNAs to determine whether PAK4 knockdown affects cell migration and displacement in pancreatic cancer cells where PAK4 is overexpressed (FIG. 3A).

When PAK4 knockdown was applied to Su8686, PANC1, and PATU-8988T cells, a dramatic decrease in single cell migration was observed (FIG. 3B), and a pattern of inhibiting group cell migration was confirmed (FIG. 3C). In addition, the cell invasion capacity of each of the Su8686 and PANC1 cells was significantly reduced by PAK4 knockdown, compared to shCTRL (FIG. 3D).

Since cell migration and invasion are closely related to the cell displacement, the displacement of each of PANC1 shCTRL and shPAK4 cells was evaluated, and it was confirmed that the displacement of the shPAK4 cells was statistically significantly reduced (FIG. 3E). In addition, it was confirmed that PANC1 shCTRL cells have a metastatic protruding three-dimensional structure, whereas shPAK4 shows small spherical characteristics when cultured three-dimensionally, and the metastatic properties were lowered during the PAK4 knockdown (FIG. 3F).

### [Embodiment 4] Analysis of Effect of PAK4 Knockdown on EMT-Regulating Transcript and Protein Expression

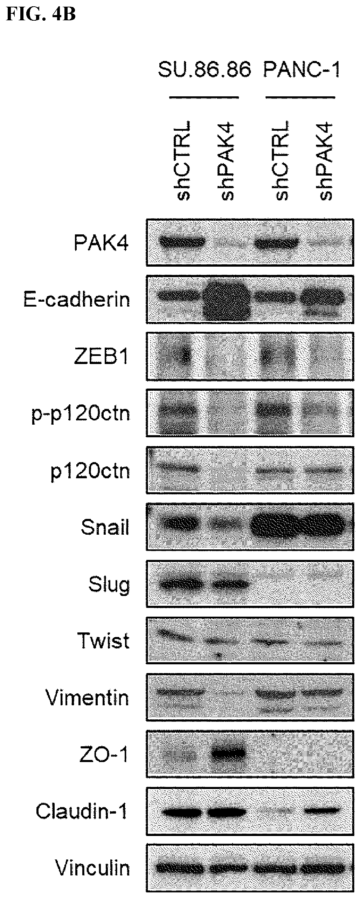

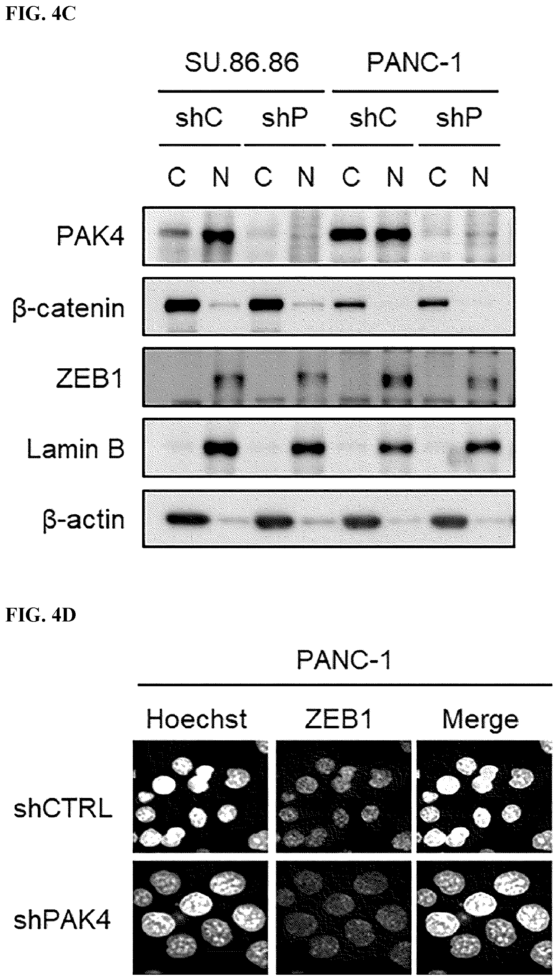

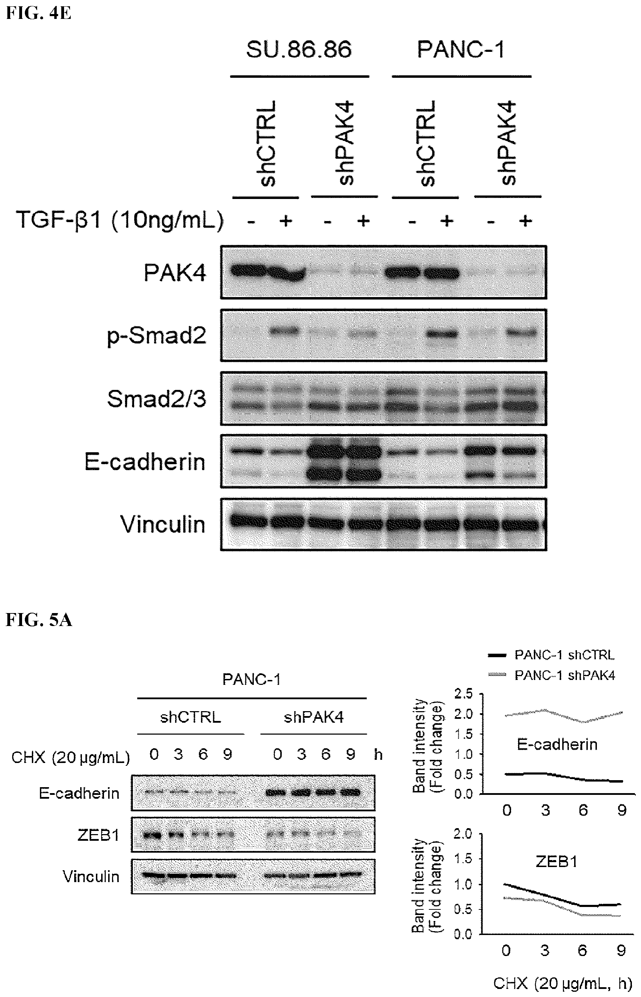

**Experiment Method:** RNA of SU.86.86, PANC1 shCTRL or shPAK4 was used to analyze mRNA expression of EMT-related genes by Realtime RT-PCR analysis (FIG. 4A). EMT-related proteins expressed in SU.86.86, PANC1 shCTRL or shPAK4 cells were analyzed (FIG. 4B). Proteins in SU.86.86, PANC1 cells were separated into cytoplasm and nucleus to evaluate β-catenin, ZEB1, E-cadherin Lamin B, β-actin, and the like (FIG. 4C). Intracellular expression of ZEB1 was analyzed by immunofluorescence in PANC-1 cells, and the scale bar is 20 µm (FIG. 4D). SU.86.86 and PANC-1 cells were treated with 10 ng/mL TGF-β for 24 hours, and then expressions of PAK4, p-Smad2, Smad2/3, and E-cadherin were analyzed by immunoblotting (FIG. 4E).

**Experiment Result:** There are various signal transmission mechanisms that lead to metastasis from primary cancer, but the most frequently observed one is the EMT. During this process, the expression of epithelial phenotypic factor, E-cadherin, decreases, and the expression of genes including mesenchymal phenotypic factors, Zeb1, Zeb2, Snail, Slug, Twist, and Vimentin, increases.

In this experiment, it was confirmed that PAK4 knockdown in Su.86.86 and PANC1 cells increased the protein expression and transcript of epithelial phenotypic factor, E-cadherin, which is a factor involved in EMT, and decreased the expression of mesenchymal phenotypic factors (FIGS. 4A and 4B).

In addition, in this experiment, it was evaluated whether the mechanism by which PAK4 regulates E-cadherin is dependent on β-catenin, ZEB1, and TGF-β1 (FIGS. 4C, 4D, and 4E), but it was confirmed that there was no significant relationship with these factors.

### [Embodiment 5] Analysis of Effect of PAK4 on Transcription and Translation of E-cadherin

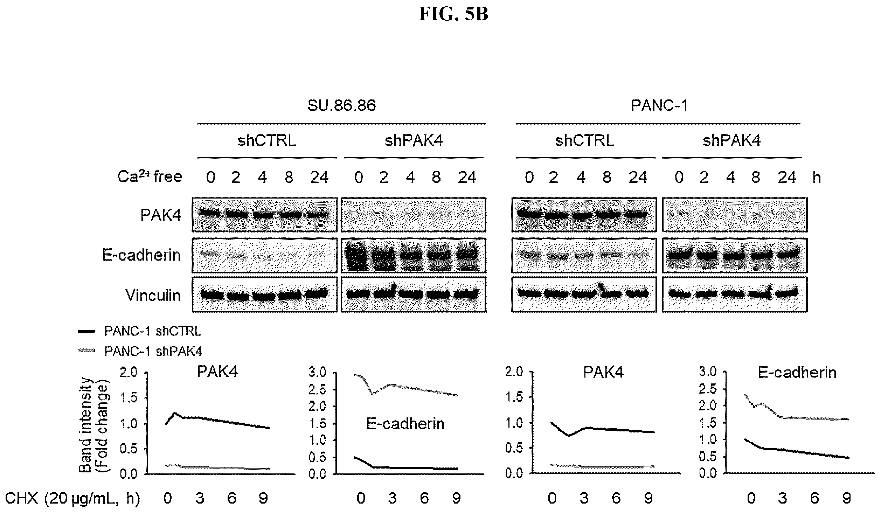

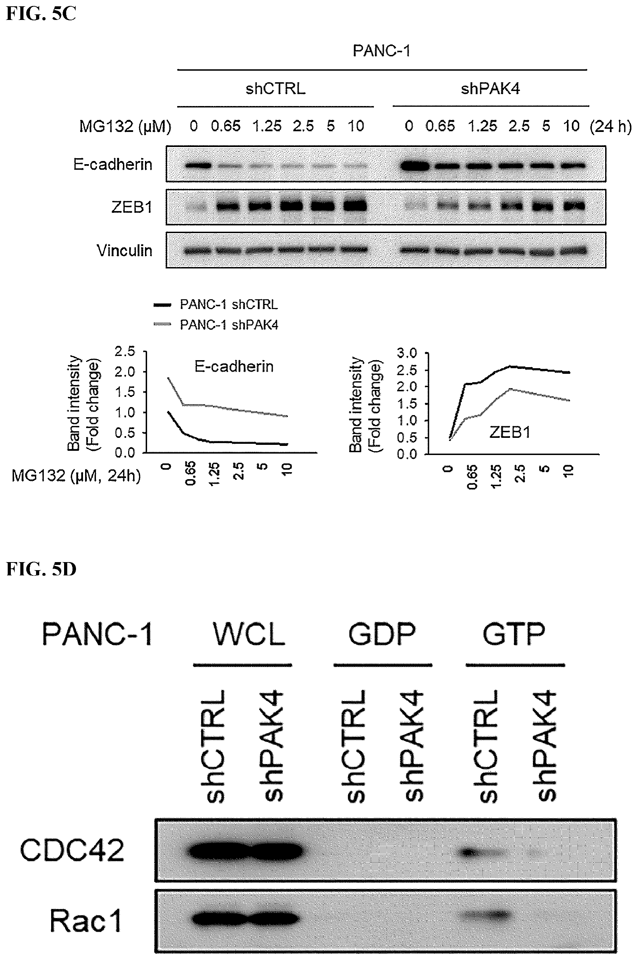

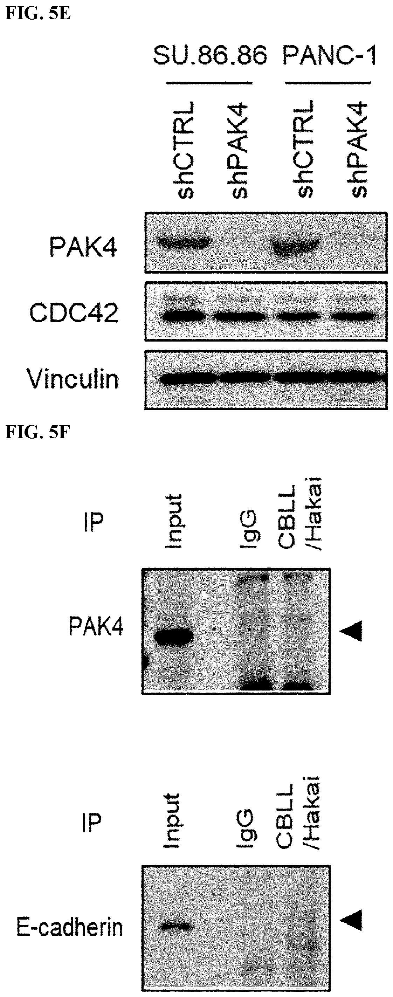

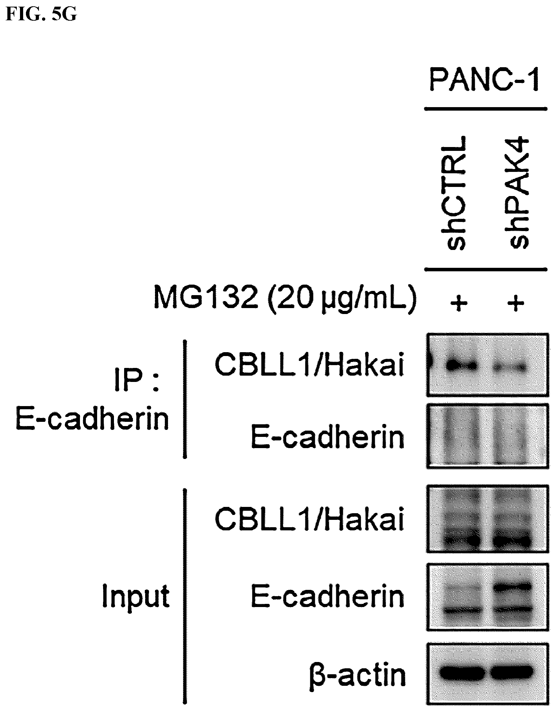

**Experiment Method:** PANC-1 cells were treated with 20 µg/mL cycloheximide (CHX), harvested at designated time points, and detected by western blot for E-cadherin, ZEB1, and Vinculin antibodies (left side of FIG. 5A). Quantitative evaluation of E-cadherin and ZEB1 protein levels was performed (normalized by Vinculin, right side of FIG. 5A). SU.86.86 and PANC-1 cells were cultured overnight in 1% P/S/10% FBS/DMEM, then converted to DMEM without Ca2+, and cultured for a specific time. E-cadherin, ZEB1, and Vinculin were detected by western blot (top side of FIG. 5B). E-cadherin and ZEB1 protein levels were quantitatively assessed (normalized by Vinculin, bottom side of FIG. 5B). PANC-1 cells stably expressing shCTRL or shPAK4 were treated with specific concentrations of MG132 for 24 hours and then detected by western blot for E-cadherin, ZEB1, and Vinculin antibodies (left side of FIG. 5C). E-cadherin and ZEB1 protein levels were quantitatively assessed (normalized by Vinculin, right side of FIG. 5C). After PANC-1 cells stably expressing shCTRL or shPAK4 were cultured in a serum-free medium for a certain period of time, activated Cdc42 and Rac1 interacting with GST-PBD-agarose were harvested from whole cell proteins. The activity of Cdc42 and Rac1 was detected by western blot (FIG. 5D). The western blot was performed to detect PAK4 and Cdc42 levels in SU.86.86 and PANC-1 cells, and the protein expression was normalized by Vinculin (FIG. 5E). To confirm the interaction between PAK4 and CBLL1/Hakai in SU.86.86 cells, cells were treated with the serum-free medium for 24 hours, followed by immunoprecipitation for anti-CBLL1/Hakai, followed by western blot with the indicated antibodies (FIG. 5F). Proteins immunoprecipitated with E-cadherin antibodies were subjected to western blot for CBLL1/Hakai and E-cadherin in proteins of PANC-1 cells. The western blot was performed with the indicated antibodies using the whole cell proteins of PANC-1 (FIG. 5G).

**Experiment Result:** When evaluating whether E-cadherin due to PAK4 knockdown is regulated by transcription, it was confirmed that E-cadherin production and ZEB1 production were inhibited by cyclohexamide in shCTRL cells, but the E-cadherin expression remained the same and only the ZEB1 expression was significantly reduced in the PAK4 knockdown, thereby confirming that PAK4 stabilizes the E-cadherin protein independently of ZEB 1 (FIG. 5A).

In addition, as a result of confirming whether PAK4 affects the E-cadherin translation process, E-cadherin in shCTRL decreased in a time-dependent manner when there is no calcium in SU.86.86 and PANC-1 cells. However, in PAK4 knockdown, the E-cadherin expression did not decrease, and the same pattern was observed in the experiment in which MG132 was treated (FIGS. 5B and 5C). Furthermore, when MG132 was treated, the amount of protein of E-cadherin in shCTNL decreased in a concentration-dependent manner, but such a pattern was not observed in PAK4 knockdown. This result can be interpreted that the increase in E-cadtherin expression due to PAK4 knockdown is independent of ZEB1.

On the other hand, protein stability of E-cadherin is known to be regulated by CDC42 GTP activity, and it has been reported that PAK4 binds to CDC42 and regulates the function of CDC42. In addition, proteolysis of E-caedherin has been reported to be specifically degraded by CBLL1/Hakai ubiquitinase.

As a result of confirming whether the increased E-cadherin expression due to PAK4 knockdown was caused by CDC42 GTP activity or CBLL1/Hakai interaction (FIGS. 5D, 5E, and 5F), the CDC42 GTP activity was significantly lower in PAK4 knockdown than in the shCTRL group, and there was no difference in total protein expression of CDC42. In addition, it was confirmed that PAK4 and E-cadherin bind to CBLL1/Hakai (FIG. 5F).

In addition, when CBLL1/Hakai was western blotted to protein harvested from shCTRL and PAK4 knockdown with E-cadherin antibodies (FIG. 5G), it was confirmed that the expression of CBLL1/Hakai was lower in PAK4 knockdown than in shCTRL. This result can be interpreted that PAK4 knockown prevents the binding of E-cadherin and CBLL1/ Hakai.

### [Embodiment 6] Colocalization and Binding Analysis of PAK4, CDC42, and E-cadherin

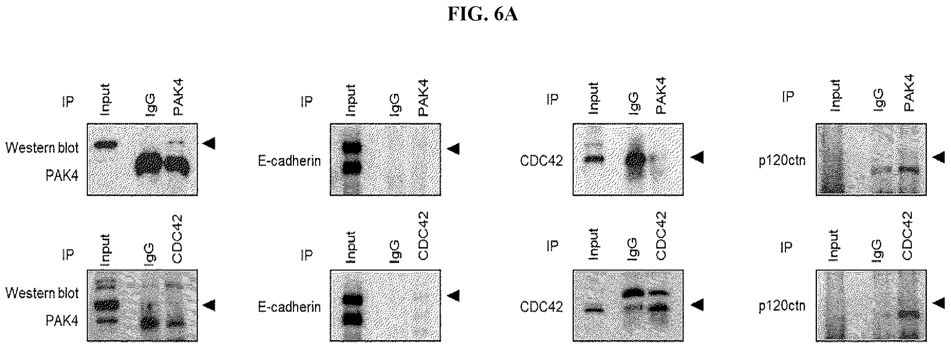

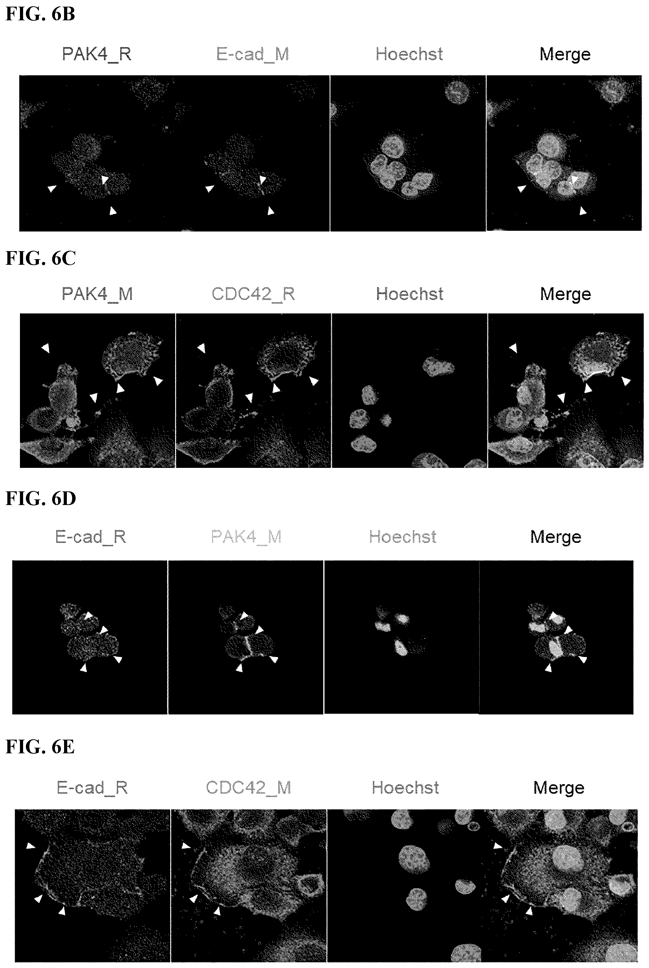

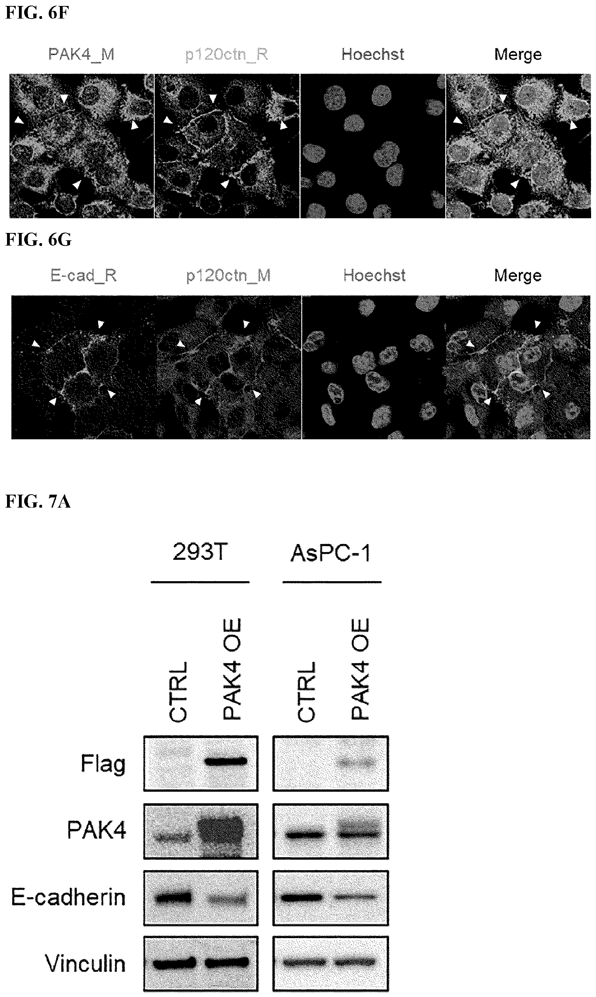

**Experiment Method:** Protein was harvested by immunoprecipitated PAK4, Cdc42 or control IgG antibodies in SU.86.86 cells. The protein immunoprecipitated by PAK4 or Cdc42 were then subjected to western blot for PAK4, E-cadherin, Cdc42, and p120ctn (FIG. 6A). PAK4 (green) and E-cadherin (red) were found to coexist in SU.86.86 cells (yellow in the merged image). Triple merging of PAK4, E-cadherins, and hoechst was confirmed. The size of bars consistent with the same correspond to 100 µm (FIG. 6B). PAK4 (green) and Cdc42 (red) were confirmed to coexist in SU.86.86 cells (yellow in the merged image, FIG. 6C). E-cadherin (green) and PAK4 (red) coexisted in SU.86.86 cells (yellow in the combined image, FIG. 6D). E-cadherin (green) or Cdc42 coexisted in SU.86.86 cells (yellow in the combined image, FIGS. 6E). PAK4 (green) and p120ctn (red) coexisted in SU.86.86 cells (yellow in the combined image, FIG. 6F). E-cadherin (green) and p120ctn (red) co-existed in SU.86.86 cells (yellow in the combined image; FIGS. 6G).

**Experiment Result:** In this experiment, it was confirmed that the proteins "PAK4-E-cadherin", "PAK4-CDC42", "PA K4-p120ctn", "CDC42-PAK4", "CDC42-E-cadherin", and "CDC42-p120ctn" bind to each other (FIG. 6A), and it was found that these proteins are expressed at the same position in the cells (FIGS. 6B, 6C, 6D, 6E, 6F, and 6G).

### [Embodiment 7] Experiments for Analysis of Cell Migration, Invasion, and Displacement Due to Increase in PAK4 Overexpression and CDC42 Activity, and Decrease in E-cadherin

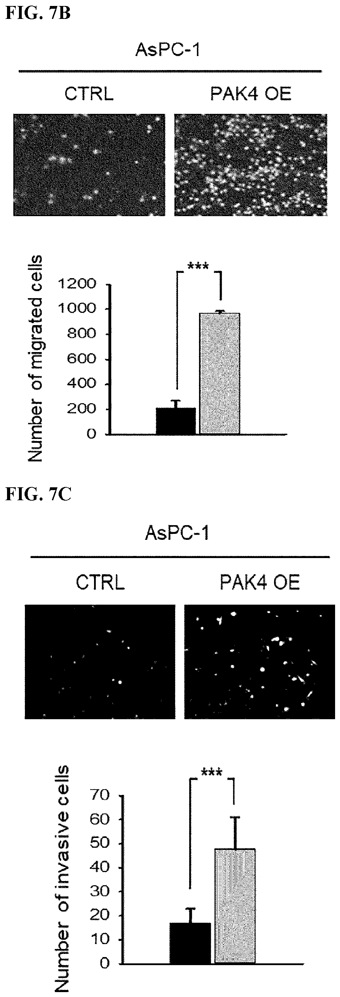

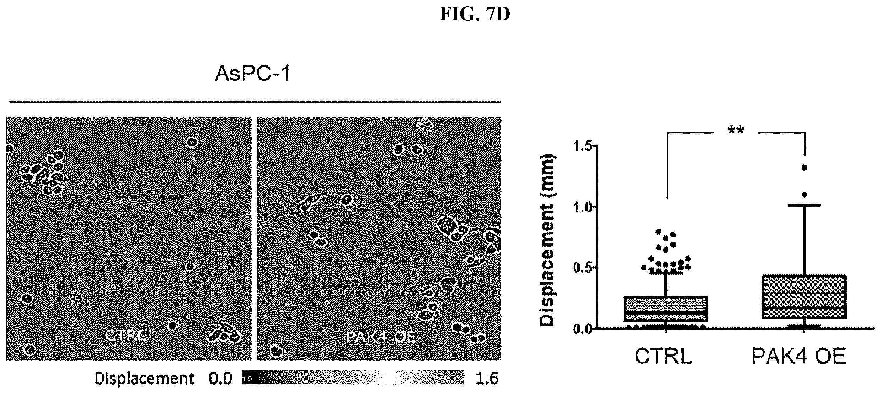

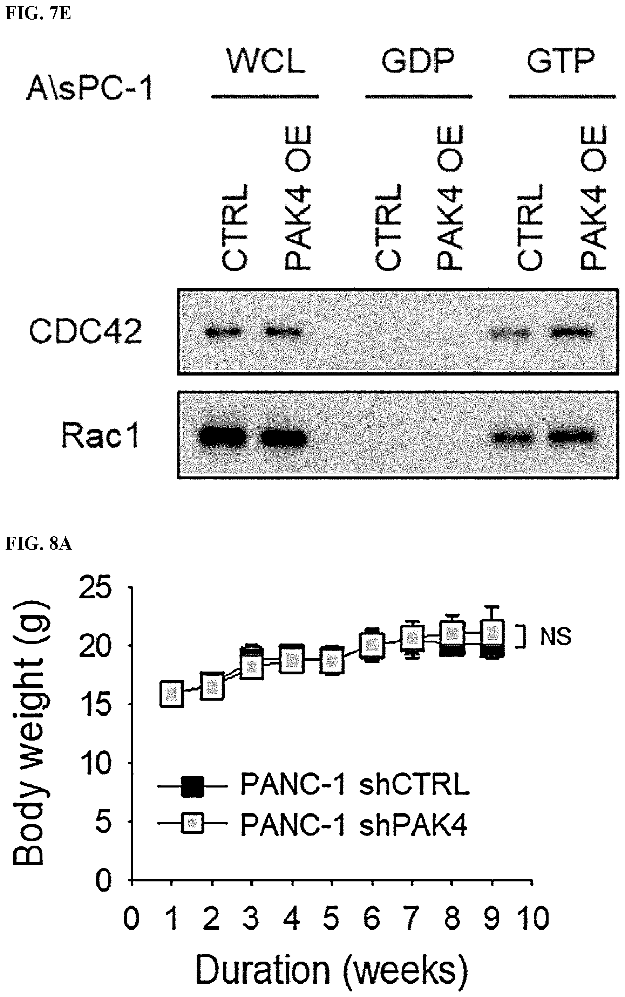

**Experiment Method:** The protein expression levels of Flag, PAK4, and E-cadherin were evaluated in 293T and AsPC-1 cells using control or PAK4-overexpressing plasmids (FIG. 7A). The effect of PAK4 overexpression on single cell migration in AsPC-1 cells was evaluated (FIG. 7B). Matrigel-coated transwell analysis was used to evaluate the effect of PAK4 overexpression on invasion of AsPC cells (FIG. 7C). The cell displacement was analyzed by IncuCyte imaging in control or AsPC-1 cells with PAK4 overexpression (FIG. 7D). The control or AsPC-1 cells with PAK4 overexpression were cultured in the serum-free medium for the indicated times, and activated Cdc42 and Rac1 were cultured with GST-PBD-agarose to evaluate the activity of affinity-precipitated Cdc42 or Rac1 from whole cell lysates (FIG. 7E).

**Experiment Result:** The results of the study in FIG. 7 show that PAK4-CDC42-E-cadherin signal transmission mechanism acts on PAK4 knockdown to reduce cell migration, invasion, displacement and the like. If this result is true, it is believed that PAK4 overexpression in PAK4 wild-type cells will result in the opposite pattern to PAK4 knockdown, thereby confirming the following experiment.

Therefore, in this experiment, in order to prove this correlation, PAK4 wild-type 293T cells and pancreatic cancer ASPC-1 cells were induced for PAK4 overexpression using genetic engineering techniques. As a result, it was confirmed that the E-cadherin expression was lowered during PAK4 overexpression in both 293T and ASPC-1 cells (FIG. 7A), and it was observed that ASPC1 cells where PAK4 is overexpressed had statistically significantly lower cell migration and invasion functions than CTRL (FIGS. 7B and 7C).

In addition, ASPC-1 cells where PAK4 is overexpressed were found to have a very significant increase in displacement, compared to CTRL cells (FIG. 7D). It was demonstrated that PAK4 overexpression caused an increase in CDC42 activity (FIG. 7E).

### [Embodiment 8] Evaluation of Effect of PAK4 Knockdown on Growth and Metastasis of Primary Cancer in Pancreatic Cancer orthotopic Model

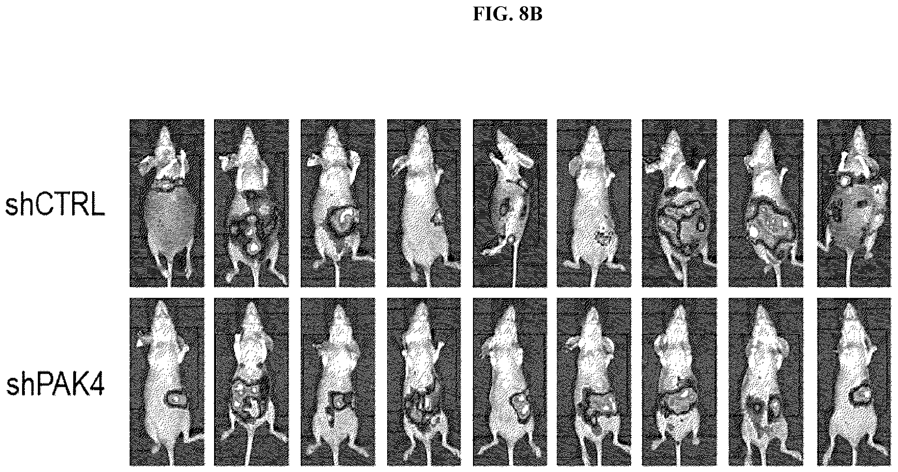

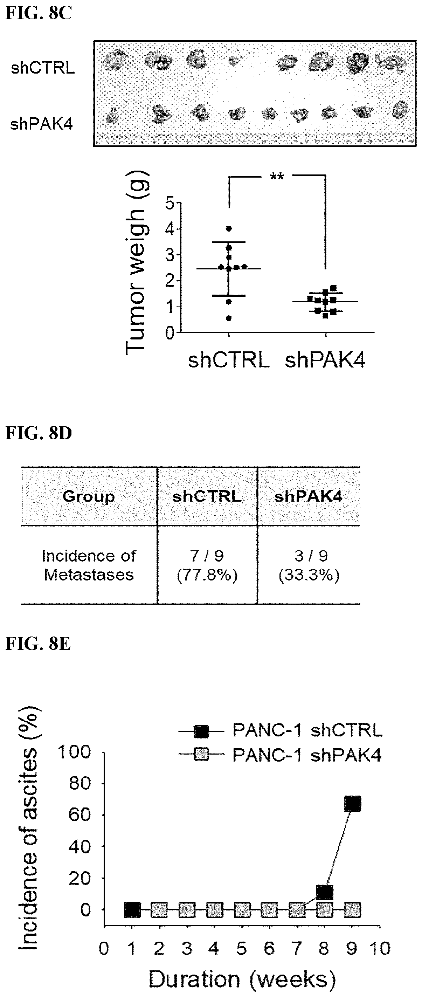

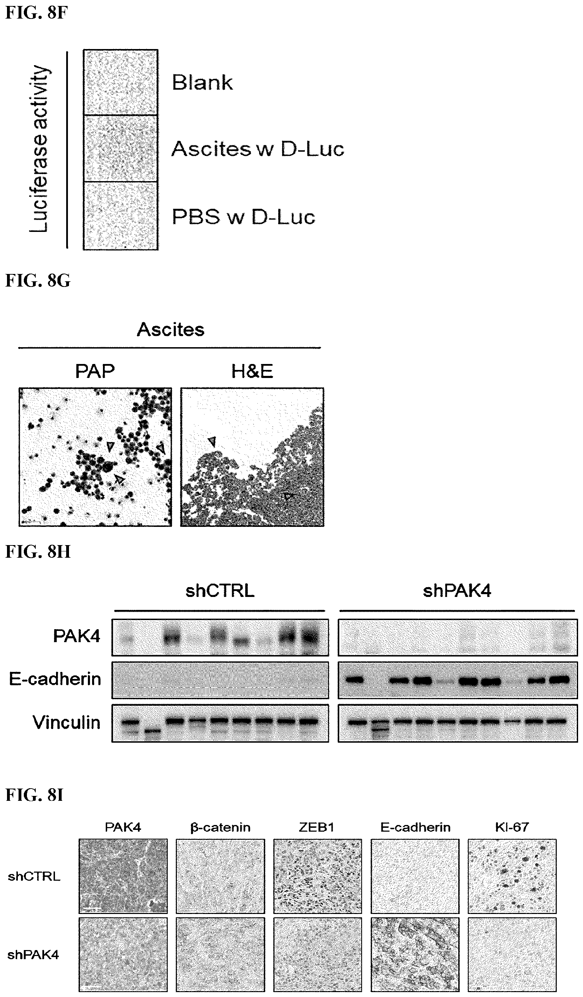

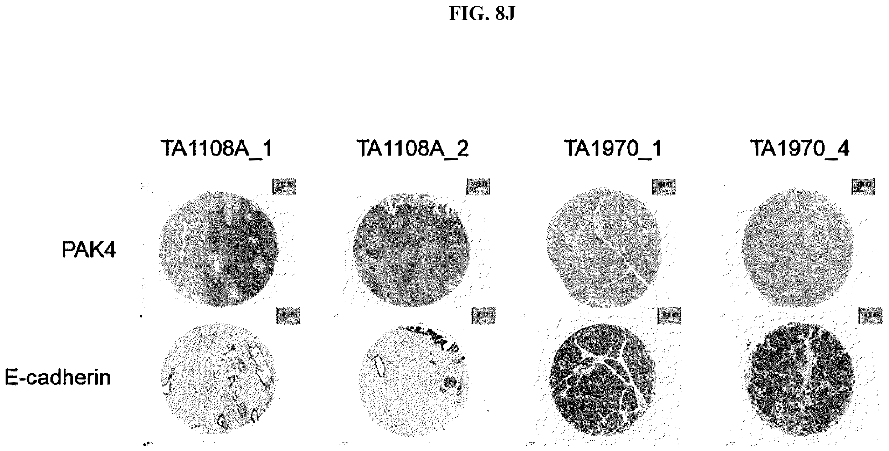

**Experiment Method:** The change in body weight for each group for 9 weeks was shown in graphs. For in vivo studies, values were expressed as mean ± standard error of the mean. NS indicates that there is no statistically significant difference (FIG. 8A). The growth of primary cancer was confirmed using IVIS200 at 9 weeks after injection of 1×10⁷ cells PANC-1 shCTRL and shPAK4 cells (FIG. 8B). Pancreatic tumors of mice were dissected and weighed (**, P<0.01; n=10, FIG. 8C). The amount of ascites was expressed (FIG. 8D), and luminescence analysis was performed on cells harvested from ascites (FIG. 8E). Papanicolaou (PAP) and H&E staining were used to validate cancer cells derived from ascites. The western blot analysis and comparison of PAK4 and E-cadherin in primary pancreatic tumors harvested from mice were performed (FIG. 8G). Pancreatic tumor sections were immune-stained using PAK4, E-cadherin, and Ki-67 antibodies according to shCTRL or shPAK4 (FIG. 8H). Immunohistochemical staining for PAK4 and E-cadherin was performed on tissue microarrays composed of excision specimens from PDAC patients (FIG. 8I).

**Experiment Result:** The above in vitro experiment result may suggest that when PAK4 is inhibited in the PAK4 overexpression model, cell migration, invasion, and displacement can be controlled to inhibit metastasis.

Therefore, in this experiment, an in vivo experiment was performed to clearly confirm the effect of PAK4 knockdown on metastasis using the pancreatic cancer orthotopic model.

As shown in FIG. 8A, there was no difference in body weight between the PANC1 shCTRL and shPAK4 groups, but the size of primary cancer in the shCTRL group grew significantly greater than that in the shPAK4 group (FIGS. 8B and 8C), and ascites (which considered metastatic in the clinic) occurred in 66.7% of animals in the shCTNL group at 8 to 9 weeks (FIG. 8D).

In order to confirm the presence of cancer cells in the ascites, the luciferase activity, fibroblast activated protein (FAP), hematoxylin and eosin (H&E), which are expressed during tissue remodeling when cancer cells grow, were analyzed, and it was confirmed that cancer cells were present in ascites in the shCTRL group (FIGS. 8E to 8F).

PAK4 knockdown was well maintained in the shPAK4 group (FIGS. 8G and 8H), and a negative correlation with E-cadherin was confirmed. In addition, KI-67, a major cancer cell growth factor, was increased in the shCTRL group, but it was confirmed that it was expressed very low in the shPAK4 group.

In addition, a negative correlation between PAK4 and E-cadherin was confirmed in the tumor microarray (TMA) of pancreatic cancer patients who visited Seoul National University Bundang Hospital (FIG. 8I), and the analysis result was obtained, which indicates that there is a possibility that metastasis can be suppressed when PAK4 is targeted.

## Claims

1. A method for screening a p21-activated kinase 4 (PAK4) inhibition and E-cadherin expression inducer, the method comprising:
treating a cell where PAK4 is overexpressed with a candidate substance;
measuring the amount of E-cadherin expression in the cells where PAK4 is overexpressed after the candidate substance treatment; and
comparing the measured amount of E-cadherin expression with the amount of E-cadherin expression before the candidate substance treatment.

2. The method of claim 1, further comprising, after the comparing step, determining that the candidate substance is PAK4 inhibition and E-cadherin expression inducer if the measured amount of E-cadherin expression is greater than the amount of E-cadherin expression before the candidate substance treatment.

3. The method of claim 1, wherein the cells where PAK4 is overexpressed are cancer cells.

4. The method of claim 3, wherein the cancer cells are pancreatic cancer cells.

5. The method of claim 1, wherein the cells where PAK4 is overexpressed are metastatic cancer cells.

6. The method of claim 5, wherein the metastatic cancer cells are derived from pancreatic cancer cells.

7. The method of claim 1, wherein the candidate substance is an anticancer drug.

8. The method of claim 7, wherein the anticancer drug is a small molecule drug.

9. The method of claim 1, further comprising overexpressing PAK4 in PAK4 wild-type cells before the candidate substance treatment.

10. The method of claim 1, further comprising:
measuring the amount of cell division cycle 42 (CDC42) expression in the cells where PAK4 is overexpressed after the candidate substance treatment and comparing the same with the amount of CDC42 expression before the candidate substance treatment; and
if the measured amount of CDC42 expression in the cells is less than the amount of CDC42 expression before the candidate substance treatment, determining that the candidate substance is the PAK4 inhibition and E-cadherin expression inducer.

11. The method of claim 1, further comprising:
measuring a change in the displacement of the cells where PAK4 is overexpressed after the candidate substance treatment and comparing the same with the displacement thereof before the candidate substance treatment; and
determining that the candidate substance is the PAK4 inhibition and E-cadherin expression inducer if the measured amount of cell displacement is less than the displacement before the candidate substance treatment.

12. The method of claim 1, further comprising:
measuring a change in the amount of gene expression in the cells where PAK4 is overexpressed, by one or more of Zeb1, Zeb2, Snail, Slug, Twist and Vimentin, after the candidate substance treatment and comparing the same with the amount of gene expression before the candidate substance treatment; and
if the measured amount of gene expression in the cells is less than the amount of gene expression before the candidate substance treatment, determining that the candidate substance is the PAK4 inhibition and E-cadherin expression inducer.

13. A composition for screening p21-activated kinase 4 (PAK4) inhibition and E-cadherin expression inducer.

14. The composition of claim 13, wherein the PAK4 inhibition and E-cadherin expression inducer is an anticancer drug.

15. The composition of claim 13, wherein the composition comprises a substance that detects E-cadherin and/or cell division cycle 42 (CDC42) or measures the amount of expression thereof.

16. The composition of claim 13, wherein the composition comprises cells where PAK4 is overexpressed.

17. A kit for screening p21-activated kinase 4 (PAK4) inhibition and E-cadherin expression inducer comprising the composition of any one of claims 13 to 16.

18. A pharmaceutical composition for preventing or treating cancer, comprising p21-activated kinase 4 (PAK4) inhibition and E-cadherin expression inducer as an active ingredient.

19. The pharmaceutical composition of claim 18, wherein the PAK4 inhibition and E-cadherin expression inducer increases the amount of E-cadherin expression in cells where PAK4 is overexpressed.

20. The pharmaceutical composition of claim 18 or 19, wherein the PAK4 inhibition and E-cadherin expression inducer is a PAK4 inhibition, CDC42 inhibition and E-cadherin expression inducer that reduces the amount of cell division cycle 42 (CDC42) expression in cells where PAK4 is overexpressed.

21. The pharmaceutical composition of claim 18, wherein the cancer is pancreatic cancer.

22. The pharmaceutical composition of claim 18, wherein the cancer is metastatic cancer.

23. The pharmaceutical composition of claim 18, wherein the pharmaceutical composition is a cancer metastasis inhibitor that inhibits cancer metastasis.

24. The pharmaceutical composition of claim 18, wherein the PAK4 inhibition and E-cadherin expression inducer is a low molecular weight compound.
